# EUROPÄISCHE PATENTSCHRIFT

(11) **EP 0 873 761 B1**
(45) Veröffentlichungstag und Bekanntmachung des Hinweises auf die Patenterteilung: **10.11.2004**
(21) Anmeldenummer: 98105235.0
(22) Anmeldetag: 23.03.1998
(51) Int. Cl.: A61M 25/01, A61B 1/31

(54) **Stülpschlauchsystem**
Verting sleeve system
Système à manchon inversé

(30) Priorität: 23.04.1997 DE 19717108
(43) Veröffentlichungstag der Anmeldung: 28.10.1998
(60) Teilanmeldung: 04006220.0
(73) Patentinhaber: STM Medizintechnik Starnberg GmbH, 86947 Schwabhausen/Weil (DE)
(72) Erfinder: Viebach, Thomas, 82282 Pischertshofen (DE); Pauker, Fritz, 86316 Wiffertshausen/Friedberg (DE); Buchmann, Gerhard, 82335 Berg (DE); Weiglhofer, Gerhard, 86947 Schwabhausen/Weil (DE); Pauker, Robert, 86438 Kissing (DE)
(74) Vertreter: Leson, Thomas Johannes Alois, Dipl.-Ing.

(56) Entgegenhaltungen:
- WO-A-85/00097
- DE-A- 2 406 823
- DE-A- 4 242 291

## Beschreibung

Die Erfindung betrifft die Konstruktion eines Stülpschlauchsystems vorzugsweise für ein Endoskopiegerät zur Untersuchung von kanalartigen Hohlräumen zum Beispiel am menschlichen Körper gemäß dem Oberbegriff des Patentanspruchs 1.

Endoskope werden im wesentlichen zur visuellen Untersuchung der Speiseröhre, des Magens, des Darms vom Magen oder vom Anus aus, der Harnröhre sowie der Blase eingesetzt. Hierfür ist das Endoskop an seinem distalen Ende mit einer Beleuchtungseinrichtung sowie mit einer Optik vorzugsweise einem Kamerachip ausgestattet, der über Leitungen innerhalb eines Endoskopschafts mit einer Kamerasteuerung am Ende des Endoskopschafts verbunden ist. Die Kamerasteuerung ist wiederum über einen Videoprozessor mit einem externen Monitor verbunden, auf dem der behandelnde Arzt die zu untersuchenden Bereiche erkennen kann. Das in den Hohlraum einzuführende distale Ende des Schafts ist dabei in jede Richtung abkrümmbar ausgebildet und läßt sich mittels einer Handhabe, vorzugsweise über zwei Steuerräder mit Bremse am hinteren Endabschnitt des Endoskops manuell ähnlich eines Fingers abwinkeln. Des weiteren ist in der Regel der Endoskopschaft mit zumindest zwei Kanälen durchzogen, die sich an der vordersten Spitze des distalen Endes öffnen. Durch diese Kanäle kann bei Bedarf einmal Reinigungsfluid zum Säubern einer zu untersuchenden Stelle oder Co2 (Luft) zum Entfalten des Hohlraums gepreßt oder zum Ändern über einen Arbeitskanal diverse Arbeitsgerätschaften beispielsweise Zangen oder Scheren zur Entnahme von Gewebeproben, Biopsienadeln, aufheizbare Schneiddrähte, Koagulationselektroden geschoben werden, die am hinteren Ende des Endoskopschafts ebenfalls über Bedienungsdrähte oder Bowdenzüge innerhalb des inneren Kanals manuell betätigt werden können. Bei einer Gewebeprobeentnahme wird nachdem das distale Ende die betreffende Stelle erreicht hat beispielsweise eine Zange vom hinteren Abschnitt des Endoskopschafts aus in den Kanal eingeführt und zum distalen Ende vorgeschoben. Nach erfolgter Entnahme der Probe wird die Zange wieder zurückgezogen und aus dem Kanal entfernt, so daß mit der weiteren Untersuchung fortgefahren werden kann.

Im allgemeinen hat das Endoskop eine langgestreckte schlauchförmige Gestalt mit einem Durchmesser von ca. 9 bis 15 mm und besteht aus einem biegsamen Material, um Krümmungen des zu untersuchenden Hohlraums, beispielsweise Darmwindungen folgen zu können.

Aus dem Stand der Technik beispielsweise gemäß der DE 42 42 291 A1 ist nunmehr ein Endoskop dieser Gattung bekannt.

Dieses Endoskop besteht im wesentlichen aus einem Endoskopkopf oder distalen Ende, an das sich ein Endoskopschaft aus einem flexiblen biegsamen Rohrkörper anschließt und einer Bedienungseinrichtung am hinteren Ende des Endoskopschafts. Die Bedienungseinrichtung hat eine Anzahl von drehbar am Endoskopschaft gelagerten Betätigungsrädern, die über Bedienungsdrähte oder Bowdenzüge, welche innerhalb des Endoskopschafts verlegt sind, mit dem distalen Ende wirkverbunden sind. Ferner ist in einem hinteren Endabschnitt des Endoskops eine erste Antriebs- oder Vorschubeinrichtung vorgesehen, die über Antriebsräder eine Antriebskraft auf den Endoskopschaft ausübt.

Um den Endoskopschaft ist zumindest in dessen vorderem Abschnitt ein Stülpschlauch angeordnet, der durch eine zweite Antriebs- oder Vorschubeinrichtung angetrieben wird. Der Stülpschlauch besteht dabei aus einem inneren Schlauchabschnitt, der gleitfähig an der Mantelfläche des Endoskopschafts anliegt und im Bereich des distalen Endes des Endoskops zu einem vorderen äußeren Schlauchabschnitt umgestülpt ist. Der vordere äußere Schlauchabschnitt ist ferner bis zur zweiten Antriebseinrichtung zurückgeführt und an deren Gehäuse fixiert. Im hinteren Bereich des Endoskops ist der innere Schlauchabschnitt zu einem hinteren äußeren Schlauchabschnitt umgestülpt, der ebenfalls zur zweiten Antriebseinrichtung zugeführt und an deren Gehäuse an der zum vorderen äußeren Schlauchabschnitt gegenüberliegenden axialen Endseite fixiert ist.

Die zweite Antriebseinrichtung wirkt dabei auf den inneren Stülpschlauchabschnitt, um diesen in Axialrichtung des Endoskopschafts zu bewegen. Hierfür hat die zweite Antriebseinrichtung eine Art Manschette oder Kragen, die sich in Radialrichtung zusammenziehen und dabei reibschlüssig an den inneren Schlauchabschnitt anpressen und ferner in Axialrichtung des Endoskops hubkolbenförmig bewegen läßt. Die radial wirkende Anpreßkraft der Manschette ist dabei so groß gewählt, daß zumindest ein Teil der aufgebrachten Anpreßkraft durch eine Materialverformung des inneren Schlauchabschnitts auf die Mantelfläche des Endoskopschafts übertragen wird, sodaß der Endoskopschaft zusammen mit dem inneren Stülpschlauch angetrieben wird.

Da bei dieser alleinigen Antriebsart durch die zweite Antriebseinrichtung die Vorschubgeschwindigkeit (und Strecke) des Stülpschlauchs an seinem vorderen Stülpbereich aufgrund dessen Stülpbewegung nur halb so groß wäre, wie die des Endoskopschafts, d.h. der Endoskopschaft würde mit zunehmender Eindringtiefe aus dem Stülpschlauch teleskopförmig herausfahren, übt die eingangs genannte erste Antriebseinrichtung eine Bremskraft auf den Endoskopschaft aus, die der Vorschubkraft der zweiten Antriebseinrichtung entgegenwirkt.

Die zweite Antriebseinrichtung ist dabei zu der ersten Antriebseinrichtung synchronisiert, derart, daß im Zusammenspiel beider Antriebseinrichtungen die Bewegungsgeschwindigkeit des inneren Schlauchabschnitts in eine axiale Richtung doppelt so hoch ist wie die Bewegungsgeschwindigkeit des Endoskopschafts, wobei dieser relativ zum inneren Endoskopschaft gleitet (d.h. das distale Ende des Endoskopschafts bewegt sich mit gleicher Geschwindigkeit wie der Umstülpbereich des Stülpschlauchs).

Um die Relativgleitbewegung zwischen dem Endoskopschaft und dem Stülpschlauch zu erleichtern, sieht der Stand der Technik gemäß der DE 42 42 291 A1 ferner eine Schmiervorrichtung vor, mittels der ein Schmier- oder Gleitmittel zwischen den inneren Schlauchabschnitt und den Endoskopschaft sowie zwischen den inneren und äußeren Schlauchabschnitt einpreßbar ist. Hierfür hat die Schmiervorrichtung eine konusförmige Hülse, die über den Endoskopschaft gestreift ist und mit dem hinteren Stülpbereich des Stülpschlauchs, der auf die konusförmige Hülse aufreitet, dichtend zusammenwirkt. Das Schmiermittel, welches mittels einer Pumpe in den Spalt zwischen der konusförmigen Hülse und dem Endoskopschaft eingepreßt wird, breitet sich zwischen dem inneren Schlauchabschnitt und dem Endoskopschaft auf die gesamte Länge aus, wobei Überschußmengen des Schmiermittels im vorderen Umstülpbereich des Stülpschlauchs in den zu untersuchenden Hohlraum austreten.

Es hat sich nunmehr gezeigt, daß das Endoskop gemäß der DE 42 42 291 A1 insbesondere hinsichtlich des Antriebs des Stülpschlauchs sowie des Endoskopschafts als auch der relativ hohen Leckageverluste an Schmiermittel im vorderen Umstülpbereich des Stülpschlauchs gewisse Mängel aufweist, die den Einsatz des Endoskops erschweren.

So erweist sich insbesondere die vorstehend beschriebene zweite Antriebseinrichtung als nachteilig, da die von der Manschette auf den Endoskopschaft über den elastisch verformten Stülpschlauch großflächig übertragende Anpreßkraft zu einer erheblichen Verschlechterung der Relativ-Gleitfähigkeit des Endoskopschafts innerhalb Stülpschlauchs trotz Zuführung von Schmiermittel führt. Insofern muß durch die erste Antriebseinrichtung eine erhöhte Bremskraft auf den Endoskopschaft aufgebracht werden, um das Geschwindigkeitsverhältnis zwischen Stülpschlauch und Endoskopschaft von 2 : 1 beizubehalten. Im übrigen ist die erste Vorschubeinrichtung nur schwer und damit aufwendig mit der hubkolbenartigen Vorschubbewegung der zweiten Vorschubeinrichtung zu synchronisieren. Es liegt daher auf der Hand, daß die erste Antriebseinrichtung nicht nur leistungsstark dimensioniert sein muß und demzufolge auch groß baut sondern auch ein größerer Regelaufwand betrieben wird. Im übrigen die die Hubkolbenbewegung wenig geeignet, das distale Ende exakt an einer zu untersuchenden Stelle zu positionieren.

Es ist zwar aus einem weiteren Stand der Technik, insbesondere gemäß der DE 39 25 484 A1 bekannt, das distale Ende des Endoskopschafts zu einem pilzförmigen Kopfstück auszubilden, das eine zum Stülpbereich des Stülpschlauchs weisende konkave Dichtfläche hat, die beim Antreiben des Stülpschlauchs mit dessen Stülpbereich in dichtenden Eingriff ist. Jedoch bedingt diese Ausbildung ein Aufweiten des Endoskopschafts insbesondere des distalen Endes und damit erneut zu einer Verschlechterung der Handhabung des Endoskops.

Es ist daher die Aufgabe der vorliegenden Erfindung, ein Stülpschlauchsystem, welches u.A. für ein gattungsgemäßes Endoskop geeignet ist, derart weiterzubilden, daß dessen Funktionalität und Handhabung verbessert wird.

Diese Aufgabe wird erfindungsgemäß durch ein Stülpschlauchsystem mit den Merkmalen des Anspruchs 1 sowie eine zugehörige Antriebseinrichtung mit den Merkmalen des Anspruchs 5 gelöst.

Gemäß dem Patentanspruch 1 hat der Stülpschlauch ein Schlauchführungsteil bzw. eine Hülse aus einem steifen Material, die über den inneren Schlauchabschnitt unter Ausbildung eines Ringspalts gezogen ist und an dessen beiden axialen Endabschnitten die freien Enden äußerer Schlauchabschnitte fixiert sind, die durch Umstülpen und Zurückführen des inneren Schlauchabschnitts an zwei axial voneinander beabstandeten Stülpbereichen gebildet werden. Diese Hülse bietet nunmehr eine Aufnahme- oder Befestigüngsmöglichkeit für eine Antriebseinrichtung unmittelbar am inneren Schlauchabschnitt, so daß die äußere Abmessung des gesamten Stülpschlauchsystems kompakt bleibt und damit die Handlichkeit verbessert wird.

Es ist ferner vorgesehen, daß die Hülse in ihrem Mittenabschnitt eine Anzahl von vorzugsweise in gleichem Winkelabstand zueinander angeordneter Öffnungen oder Längsschlitze hat. Der Stülpschlauch bildet gemäß Anspruch 3 über den inneren Schlauchabschnitt, die äußeren Schlauchabschnitte sowie die Hülse einen gegenüber der Umgebung abgedichteten Hohlraum aus, der lediglich über die Öffnungen in der Hülse einen Zugang hat. Auf diese Weise wird die Hülse konstruktiv für das Anbringen einer Antriebseinrichtung vorbereitet, deren Vorschubmittel durch die Längsschlitze mit dem inneren Schlauchabschnitt in Kontakt bringbar sind. Des weiteren ist die Hülse nach Anspruch 4 mit im wesentlichen in Axialrichtung verlaufenden Nuten versehen, die sich an den Stirnflächen der Hülse öffnen. Diese Nuten erleichtern im Betrieb des Systems die zwangsläufig auftretende Verlagerung des im genannten Hohlraum befindlichen Schmiermittels durch die Hülse hindurch.

Der Erfindungsgegenstand nach Anspruch 5 sieht zur Lösung der gestellten Aufgabe vor, daß die Antriebseinrichtung des erfindungsgemäßen Stülpschlauchsystems ein zweigeteiltes, zusammenklappbares Gehäuse hat, welches manschettenförmig um die Hülse des Stülpschlauchs legbar ist und in zusammengeklapptem Zustand mit der Hülse einen nach außen dicht verschlossenen Hohlraum bildet, im welchem Reibräder untergebracht sind, die an dem Gehäuse lagernd durch das Zusammenklappen des Gehäuses durch die Öffnungen der Hülse gegen den inneren Schlauchabschnitt preßbar sind. Hierdurch wird eine kompakte, in sich geschlossene (integrierte und nicht additive) Konstruktion des Systems erreicht, wodurch die Handhabbarkeit und Funktionalität der zusammenwirkenden Teile erhöht wird.

Die Weiterbildung des Erfindungsgegenstands nach Anspruch 6 sieht weiter vor, daß die Reibräder federnd am Gehäuse gelagert sind, um eine entsprechend der Federkraft vorbestimmte oder auch justierbare Anpreßkraft auf den inneren Schlauchabschnitt aufzubringen. Gemäß Anspruch 7 können die Reibräder zusätzlich an ihren Laufflächen mit einem Antirutschbelag versehen oder ausgebildet sein.

Dabei hat sich als besonders vorteilhaft erwiesen, daß nach Anspruch 8 am Gehäuse der Antriebseinrichtung ein Anschluß für das Zuführen eines Schmiermittels ausgebildet ist, welches über die Öffnungen der Hülse in den Hohlraum des Stülpschlauchs preßbar ist. Hierdurch läßt sich zum einen der Antriebsmechanismus selbst als auch zum anderen die Relativ-Gleitbewegung der inneren und äußeren Schlauchabschnitte schmieren und damit die Reibung verringern.

Weitere vorteilhafte Ausgestaltungen sind Gegenstand der übrigen Unteransprüche.

Die Erfindung wird nachstehend anhand bevorzugter Ausführungsbeispiele unter Bezugnahme auf die begleitenden Zeichnungen näher erläutert. Es zeigen:
Fig. 1 ein Endoskop mit integrierter Schaftschmierung und Reibradantrieb gemäß einem ersten Ausführungsbeispiel der Erfindung,
Fig. 1a eine Vergrößerung der Verbindungsstelle zwischen einem Auslaßschlauch eines Schmiermittel-Druckbehälters und einem Zuführschlauch des Endoskops,
Fig. 2 ein Endoskop mit Schaftschmierung über ein hinteres Klemmstück gemäß einem zweiten Ausführungsbeispiel der Erfindung, wobei dieses mit einem Schmiermittel-Einspritzschuh verbunden ist,
Fig. 2a einen Längsschnitt des hinteren Schaftabschnitts in Vergrößerung zur Illustration der Schmiermittelzufuhr,
Fig. 2b einen Querschnitt des hinteren Schaftabschnitts von Fig. 2a,
Fig. 2c eine Variante des Ausführungsbeispiels gemäß Fig. 2, wonach das hintere Klemmstück ein Gleitteil hat, das durch eine Feder gegen den Stülpschlauchgedrückt wird, die sich wiederum an einem fixierten Teil des Klemmstücks abstützt,
Fig. 3 eine Stülpschlauchkonstruktion, wie sie beim Endoskop gemäß dem ersten und zweiten Ausführungsbeispiel Anwendung findet,
Fig. 3a eine Querschnittsansicht einer Antriebs- und Führungshülse gemäß der Schnittlinie A-A in Fig. 3,
Fig. 4 eine Vorrichtung zur Montage des Stülpschlauchs und des Endoskopschafts mittels eines Vakuumrohrs oder zweier Halbschalen und
Fig. 5 ein Endoskop gemäß einem dritten bevorzugten Ausführungsbeispiel der Erfindung.

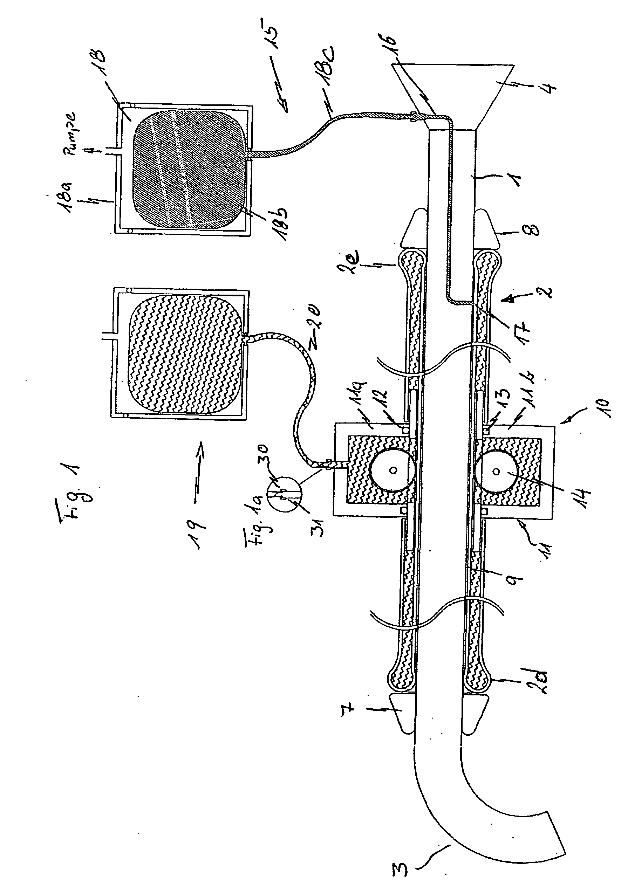

In Fig. 1 ist der schematische Seitenriß eines Endoskops gemäß einem ersten Ausführungsbeispiel der Erfindung dargestellt.

Demzufolge besteht das erfindungsgemäße Endoskop aus einem Endoskopschaft 1, der über eine Länge von 1 bis 2 m (in der Regel ca. 1600 mm) mit einem Stülpschlauch 2 der Doppelstülpbauart bzw. mit beidseitiger Umstülpung ummantelt ist. Das distale Ende 3 des Endoskopschafts 1 besteht aus einem in alle Richtungen abwinkelbaren oder krümmbaren Endstück an dessen Spitze eine Beleuchtungseinrichtung sowie ein Kamerachip angeordnet sind (beides nicht gezeigt), der über elektrische Leitungen, die innerhalb des Endoskopschafts 1 verlegt sind, an einer Kamerasteuerung am hinteren Endabschnitt des Endoskops verbunden ist. Die Kamera ist wiederum über einen Videoprozessor an einen Monitor angeschlossen.

Alternativ hierzu kann natürlich auch eine Optik an der Spitze des abwinkelbaren Endstücks 3 vorgesehen sein, das über Lichtleiter z.B. Glasfaserbündel innerhalb des Endoskopschafts 1 mit der Kamerasteuerung verbunden ist.

Im Endoskopschaft 1 erstreckt sich ferner zumindest zwei Kanäle oder Schläuche, die sich an der vordersten Spitze des krümmbaren Endstücks 3 nach außen öffnen und die am hinteren Endabschnitt des Endoskops einen Einführanschluß bzw. manuell steuerbare Ventile besitzen. Einer dieser Kanal dient der Luftoder Gaszufuhr und als Zuführkanal von Reinigungsfluid für das Spülen zu untersuchender Stellen des Hohlraums, der andere als Arbeitskanal zum Einführen von medizinischen Arbeitsgeräten, die bis zum distalen Ende vorgeschoben werden können, um beispielsweise Gewebeproben zu entnehmen oder chirurgische Eingriffe vorzunehmen.

Zur Betätigung des Endstücks 3 ist dieses über innerhalb des Endoskopschafts 1 verlegte Bowdenzüge (nicht gezeigt) mit einer manuellen Betätigungseinrichtung 4 wirkverbunden, die an einem Endabschnitt des Endoskopschafts 1 innerhalb eines Gehäuses untergebracht ist.

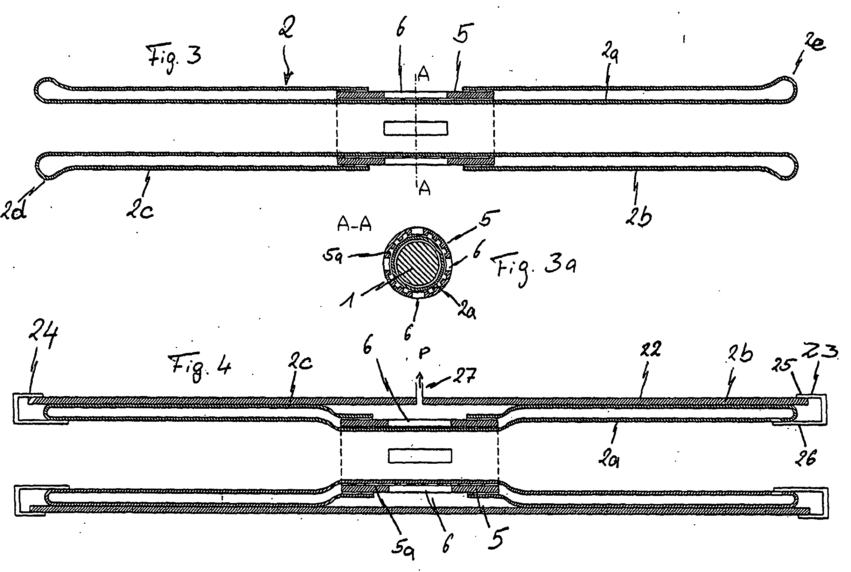

Wie insbesondere aus den Fig. 1 und 3 zu entnehmen ist, besteht der erfindungsgemäße Stülpschlauch 2 aus einem inneren Schlauchabschnitt 2a, der durch eine Antriebs- und Führungshülse bzw. ein Schlauchführungsteil 5 gleitfähig hindurchgeführt und in seinem vorderen Bereich (Umstülpbereich) zu einem vorderen äußeren Schlauchabschnitt 2b umgestülpt ist. Der vordere äußere Schlauchabschnitt 2b ist dabei zu der Antriebs- und Führungshülse (Schlauchführungsteil) 5, welche aus einem steifen Material, vorzugsweise einem Kunststoffmaterial besteht, zurückgeführt und an einem axialen Ende an der Antriebshülse 5 derart befestigt, daß diese zwischen dem inneren 2a und äußeren Schlauchabschnitt 2b zu liegen kommt.

In einem hinteren Bereich (Umstülpbereich) ist der innere Schlauchabschnitt 2a zu einem hinteren äußeren Schlauchabschnitt 2c umgestülpt, der zu der Antriebshülse 5 zurückgeführt und an einem axialen Ende der Antriebshülse 5 fixiert ist. Die Antriebshülse 5 dient zum einen als Führungselement für den inneren Schlauchabschnitt 2a, um Aufwerfungen und Falten- bzw. Schuppenbildung zu verhindern und zum anderen als Verbindungsstück des vorderen äußeren 2b und hinteren äußeren Schlauchabschnitts 2c, wobei ein Mittenbereich der Antriebshülse 5 exponiert, d.h. nicht von dem Stülpschlauch 2 überdeckt verbleibt. In diesem Mittenabschnitt weist die Antriebshülse 5 zumindest eine Öffnung, vorzugsweise einen in Axialrichtung sich erstreckenden Längsschlitz 6 vorbestimmter Breite auf. Vorliegend sind vier, im gleichen Winkelabstand zueinander angeordneter Längsschlitze 6 vorgesehen, wie insbesondere in der Fig. 3a gezeigt wird. Des weiteren zeigt die Antriebshülse 5 gemäß Fig. 3a an ihrer Innenseite eine Anzahl von durchgehenden Längsnuten 5a, die sich an den Stirnseiten der Antriebshülse 5 öffnen. Diese Längsnuten 5a können dabei entweder achsparallel oder spiralförmig gezogen sein.

Wie insbesondere aus der Fig. 1 zu erkennen ist, ist das Material, d.h. die Materialart und die Materialstärke des Stülpschlauchs 2 derart gewählt, daß sich im vorderen und hinteren Stülpbereich 2d, 2e jeweils eine wulstförmige Aufweitung infolge einer Materialanhäufung an der Umstülpung ausbildet, die im inneren Bereich zu einer vorbestimmten Einengung des Innendurchmessers des Stülpschlauchs 2 führt.

Gemäß der Fig. 1 liegt der Stülpschlauch 2 an seinem vorderen Stülpbereich 2d gleitend an einem kegelförmigen vorderen Klemmstück 7 an, das zumindest in Axialrichtung fest auf dem Endoskopschaft 1 sitzt und sich in Eindringrichtung des Endoskops konisch verjüngt. Der hintere Stülpbereich 2e des Stülpschlauchs 2 liegt ebenfalls an einem hinteren Klemmstück 8 gleitend an, das in diesem besonderen Ausführungsbeispiel genau wie das vordere Klemmstück 7 fest auf dem Endoskopschaft 1 sitzt und auch eine entsprechende Form besitzt. Das hintere Klemmstück 8 verjüngt sich jedoch konisch entgegengesetzt zur Eindringrichtung des Endoskops. An dieser Stelle sei jedoch darauf hingewiesen, daß zumindest das hintere Klemmstück 8 eine beliebige äußere Form aufweisen kann, da es nicht in den zu untersuchenden Hohlraum eingeführt wird.

Aus der Fig. 1 ist ferner deutlich zu entnehmen, daß sich zwischen dem inneren Schlauchabschnitt 2a des Stülpschlauchs 2 und dem Endoskopschaft 1 ein schmaler Ringspalt 9 ausbildet, der axial durch die beiden Wülste in den Umstülpbereichen 2d, 2e des Stülpschlauchs 2 begrenzt wird, die sich dichtend an die äußere Mantelfläche des Endoskopschafts 1 anlegen.

Um die Antriebshülse 5 ist in deren Mittenbereich eine Antriebs- oder Vorschubeinrichtung 10 angeordnet. Diese Vorschubeinrichtung 10 besteht vorliegend aus einem Gehäuse 11 vorzugsweise aus einem Kunststoff oder einer nichtrostenden Metallegierung, in welchem der nachfolgend noch beschriebene Antriebsmechanismus für den Stülpschlauch 2 gemäß dem ersten zweiten und dritten Ausführungsbeispiel der Erfindung untergebracht ist. Das Gehäuse 11 selbst besteht aus zwei schalenförmigen Gehäusehälften 11a, 11b, die an einem freien Kantenabschnitt längs der Antriebshülse 5 aufklappbar mittels eines nicht gezeigten Gelenks oder Scharniers aneinanderscharniert und an dem gegenüberliegenden freien Kantenabschnitt mittels einer ebenfalls nicht gezeigten Riegeleinrichtung verriegelbar sind. Alternativ hierzu können die Gehäusehälften 11a, 11b natürlich auch vollständig geteilt und mittels zweier Riegeleinrichtungen verbindbar sein. An den bezüglich der Antriebshülse 5 querlaufenden Seitenwandungen des Gehäuses 11 befinden sich an jeder Gehäusehälfte 11a, 11b der äußeren Querschnittsform der Antriebshülse 5 angepaßte Aussparungen, die in zusammengeklapptem und verriegeltem Zustand des Gehäuses 11 jeweils ein in sich geschlossenes Ausnehmungs- oder Öffnungsprofil entsprechend der Außenkontur der Antriebshülse 5 bilden und diese nach Außen dichtend umschließen. Die Aussparungen sowie die freien Kanten der Gehäusehälften 11a, 11b sind an ihren jeweiligen Schnitt- bzw. Kantenflächen mit Nuten 12 (nicht weiter gezeigt) versehen, in die Dichtungsstreifen 13 eingeklemmt oder geklebt sind, die sich bei Zusammenklappen der Gehäusehälften 11a, 11b dichtend an die Mantelfläche der Antriebshülse 5 anlegen, um so einen Gehäuseinnenraum dichtend abzuschließen.

Vorliegend besitzt die Antriebs- und Schlauchführungshülse 5 im Querschnitt eine kreisförmige Außenkontur entsprechend dem Kreisquerschnitt des Stulpschlauchs 2, um eine möglichst dichte und spannungsfreie Verbindung mit dem Stülpschlauch zu gewährleisten. Sie kann aber auch eine linsenförmige oder anderweitige Querschnittsform in Abhängigkeit der Konstruktion des Antriebsmechanismus aufweisen.

Dieser Antriebsmechanismus umfaßt gemäß dem ersten bevorzugten Ausführungsbeispiel der Erfindung einen nicht weiter gezeigten Antriebsmotor, der über einen Getriebezug (ebenfalls nicht dargestellt) auf eine Anzahl von Reibrädern 14 einwirkt. Diese Reibräder 14 sind an den beiden Gehäusehälften 11a, 11b jeweils derart gelagert, daß sie in zusammengeklapptem bzw. geschlossenem Zustand des Gehäuses 11 jeweils in einen Längsschlitz 6 der Antriebshülse 5 eindringen und mit einer vorbestimmten vorzugsweise über Federn einstellbaren Anpreßkraft auf dem inneren Schlauchabschnitt 2a des Stülpschlauchs 2 reibschlüssig anliegen. Vorzugsweise ist die Lauffläche jedes Antriebsrads 14 mit einem Haftbelag (Beschichtung mit Keramikteilchen oder Metallgranulat vorbestimmter Körnung) zur Erhöhung des Reibungskoeffizienten zwischen dem Stülpschlauch 2 und dem Antriebsrad 14 überzogen. Alternativ können die Reibräder 14 natürlich auch als Ganzes aus einem entsprechenden Material, beispielsweise Schleifkeramik, gefertigt sein. Die Anpreßkraft ist ferner in Abhängigkeit von dem Material und der Wandstärke des Stülpschlauchs 2 so gewählt, daß ein gleichmäßiger, im wesentlichen schlupffreier Vortrieb des Stülpschlauchs 2 über die Reibräder 14 gewährleistet ist.

Das Endoskop gemäß dem ersten bevorzugten Ausführungsbeispiel der Erfindung ist des weiteren mit einem Schmiersystem zur Schmierung der Relativ-Gleitbewegung zwischen dem Endoskopschaft 1 und dem inneren Schlauchabschnitt 2a, sowie zwischen dem inneren und äußeren Schlauchabschnitt 2a, 2b, 2c ausgerüstet, das nachfolgend beschrieben wird.

Dieses Schmiersystem umfaßt eine erste Schmiermittelförder- und zuführvorrichtung 15 mit einer Druckluftpumpe (nicht gezeigt) bzw. einem Druckluftanschluß, der an einen Druckbehälter 18 angeschlossen ist, um diesen mit Druckluft zu beaufschlagen bzw. zuzuführen. Dieser Druckbehälter 18 besitzt einen aufklapp- oder entfernbaren Deckel 18a, mittels dem eine Beund Entladeöffnung verschließbar ist. Innerhalb des Druckbehälters 18 befindet sich ein entnehmbarer bzw. auswechselbarer Kunststoff- oder Gummibeutel 18b oder alternativ ein SChmiermittelfaltenbalg, der mit dem zu verwendenden Schmiermittel gefüllt ist. Dieser Beutel oder Balg 18b hat einen Auslaßschlauch 18c, der durch eine, vorzugsweise der Anschlußstelle der Pumpe gegenüberliegende Bohrung des Druckbehälters 18 geführt ist. Die Bohrung schließt dabei dichtend am Auslaßschlauch 18c ab, wofür in diesem Ausführungsbeispiel ein Dichtungskeder an der Bohrung angebracht ist. Zusätzlich oder alternativ zu dieser Dichtung legt sich der Beutel 18 dichtend an der Druckbehälterwand an, so daß die Auslaßbohrung im wesentlichen luftdicht von der Anschlußstelle der Pumpe, welche sich vorzugsweise im Deckel 18a befindet, durch den Beutel 18b abgetrennt wird.

Am freien Ende des Auslaßschlauchs 18c ist ein Kupplungsstück 30 vorgesehen, das mit einem entsprechenden Kupplungsgegenstück 31 eines Zuführschlauchs 16 des Endoskops verbindbar ist, das aus dem hinteren Endabschnitt des Endoskopschafts 1 insbesondere aus dem Gehäuse der Betätigungseinrichtung 4 seitlich herausragt. Der Zuführschlauch oder Kanal 16 ist hierbei, wie in der Fig. 1 andeutungsweise gezeigt wird, innerhalb des Endoskopschafts 1 bis zu einer im Endoskopschaft 1 ausgebildeten Auslaßbohrung 17 im Bereich des Stülpschlauchs 2 weiterverlegt.

Die beiden Kupplungsstücke des Auslaß- 18c und des Zuführschlauchs 16 sind in der Fig. 1a schematisch dargestellt.

Demzufolge besitzt das eine Kupplungsstuck 30 einen Kupplungsflansch mit einer Überwurfmutter oder einem Bajonettverschluß, wobei die Mündung des Auslaßschlauchs 18c durch eine Membran verschlossen ist. Das andere Kupplungsgegenstück 31 hat ein der Überwurfmutter oder dem Bajonettverschluß entsprechendes Gegenelement sowie eine nadelförmig oder mit einer Schneide ausgebildeten Kanüle als äußerstes Ende des Zuführschlauchs 16. Beim Zusammenstecken der beiden Kupplungsstücke 30, 31 durchdringt die Kanüle die Membran und stellt so eine Fluidverbindung zwischen dem Auslaßschlauch 18c und dem Zuführschlauch 16 her.

Es sei darauf hingewiesen, daß die Anordnung der Kupplung 30, 31 keinesfalls am Endoskop sein muß. Das eine Kupplungsstück 30 kann beispielsweise auch in der Bohrung des Druckbehälter 18 integriert sein, wobei in diesem Fall das Gegenstück 31 unmittelbar am Beutel 18b plaziert werden kann, so daß bei Einsetzten des Beutels 18b automatisch eine Verbindung zum Endoskop hergestellt wird. In dessen ist die erste Ausführungsvariante zu bevorzugen, da hierdurch bereits bei Zusammenstecken der beiden Schläuche 18c und 16 der Auslaßschlauch 18c über seine gesamte Länge mit Schmiermittel vorab gefüllt ist und damit eine nachträgliche Entlüftung des Schmiersystems nicht mehr erforderlich ist.

Wie ferner in der Fig. 1 gezeigt ist, durchdringt der Zuführschlauch 16 die Auslaßbohrung 17, derart, daß kein Schmiermittel in das Innere des Endoskopschafts 1 auslecken kann und mündet dabei in den Ringspalt 9 zwischen dem inneren Stülpschlauchabschnitt 2a und dem Endoskopschaft 1, der durch den vorderen und hinteren Stülpbereich 2d, 2e axial fluiddicht begrenzt wird.

Alternativ zu der vorstehend beschriebenen Ausgestaltung des Schmiersystems ist es natürlich auch möglich, daß der Zuführschlauch 16 bzw. der Auslaßschlauch 18c direkt an einen Anschluß am Endoskopschaft 1 angeschlossen ist, so daß sich das zugeförderte Schmiermittel innerhalb des gesamten Endoskopschafts 1 ausbreiten kann. D.h., daß der Endoskopschaft 1 in diesem Fall selbst als Teil des Zuführkanals 16 dient, wobei das Schmiermittel über den Zuführschlauch 16 und den Endoskopschaft 1 zur Auslaßbohrung 17 gefördert wird. Dabei müssen dann sämtliche Innereien" des Endoskopschafts 1 mit einer spezielen Beschichtung oder Isolierung überzogen oder in Kanälen geführt sein, um eine Kontamination und damit verbundene Beschädigung durch das relativ aggressive Schmiermittel zu verhindern. Anstelle der zumindest einen Auslaßbohrung 17 kann dabei natürlich auch eine Perforation des Endoskopschafts 1 an der entsprechenden Stelle vorgesehen sein, wobei im Endoskopschaft 1 eine eigene Schmiermittelleitung 16 mit meheren Auslässem zur Schaftoberfläche verlegt sein kann.

Der Förderdruck der ersten Schmiermittelfördervorrichtung 15 ist so gewählt, daß der entstehende Staudruck innerhalb des Ringspalts 9 zwischen dem Endoskopschaft 1 und dem inneren Schlauchabschnitt 2a, kleiner ist als der Anpreßdruck der wulstförmigen Umstülpbereiche 2d, 2e des Stülpschlauchs 2 auf den Endoskopschaft 1. In diesem Fall wirken die Umstülpbereiche 2d, 2e zusammen mit dem Schaft 1 unmittelbar als Dichtungen, die eine Leckage an Schmiermittel verhindern. Des weiteren hat dies den Vorteil, daß diese Art von Dichtung vorerst nicht entlang des Endoskopschafts 1 oder mit entsprechendem Anpreßdruck am vorderen Endstück 7 gleiten muß sondern systementsprechend am Endoskopschaft 1 abwälzt, wodurch eine Erhöhung der notwendigen Vorschubkraft auf den inneren Schlauchabschnitt 2a zur Überwindung von Reibkräften unterbleibt. Dies wiederum führt zu einer verringerten notwendigen Anpreßkraft der Reibräder 14 auf den inneren Schlauchabschnitt 2a. Auf diese Weise wird auch gewährleistet, daß der Endoskopschaft 1 innerhalb des Stülpschlauchs 2 drehbar bleibt, um so bei anormalen Darmwindungen ein Durchfahren durch den Darm möglich zu machen.

Des weiteren umfaßt das Schmiersystem eine zweite Schmiermittelförder- und Zuführvorrichtung 19, die über einen Zuführschlauch oder Kanal 20 mit dem Gehäuse 11 der Antriebseinrichtung 10 verbunden ist. Der konstruktive Aufbau der zweiten Schmiermittelförder- und Zuführvorrichtung 19 entspricht im Prinzip dem der ersten Schmiermittelförder- und Zuführvorrichtung 15 bzw. den hierzu beschriebenen Alternativen, so daß an dieser Stelle auf die entsprechenden Textstellen verwiesen werden kann.

Das zweite Schmiermittelförder- und Zuführeinrichtung 19 ist hingegen wie bereits angedeutet, an das Antriebsgehäuse 11 angeschlossen, um das geförderte Schmiermittel in den dicht verschlossenen Innenraum des Antriebsgehäuses 11 zu pressen, wobei die Kupplung unmittelbar an dem Gehäuse 11 angeordnet ist. Dieses Schmiermittel hat ferner über die Öffnungen oder Längsschlitze 6 in der Antriebshülse 5 Zugang in den Hohlraum zwischen dem inneren und äußeren Schlauchabschnitt 2a, 2b, 2c des Stülpschlauchs 2, um somit die Relativ-Gleitbewegung zwischen dem inneren und äußeren Schlauchabschnitt 2a, 2b, 2c zu schmieren. Darüber hinaus bewirkt das Schmiermittel innerhalb des Antriebsgehäuses 11 natürlich auch eine Schmierung des Antriebsmechanismus, d.h. der Lagerung der Reibräder 14 selbst.

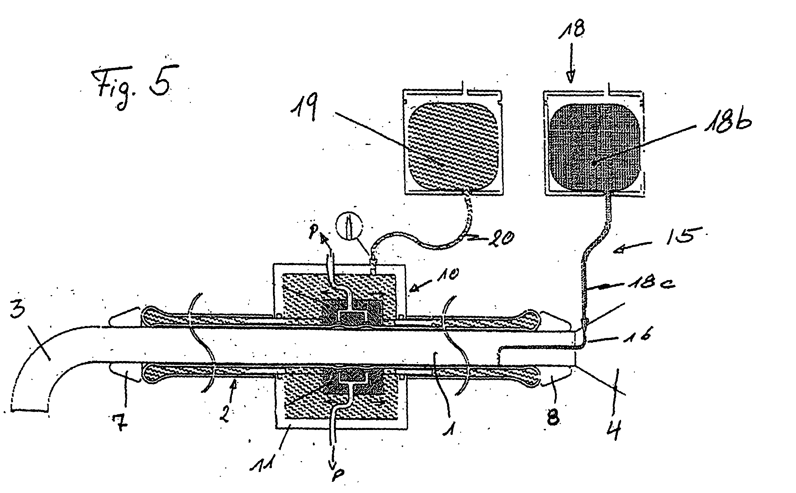

Die Betriebsbereitmachung des Endoskops gemäß dem ersten bevorzugten Ausführungsbeispiel der Erfindung wird nachfolgend anhand der Fig. 4 näher beschrieben:

Zu Beginn des Vorgangs wird über den vorgefertigten Stülpschlauch 2 ein an beiden Enden offenes Vakuumrohr 22 gezogen, dessen Innendurchmesser geringfügig größer ist als der Außendurchmesser des äußeren Schlauchabschnitts 2b, 2c. Über die Endkanten des Vakuumrohrs 22 werden anschließend zwei Dichtungsmanschetten 23, 24 gesteckt. Der Außenteil 25 jeder Dichtungsmanschette 23, 24 legt sich dabei dichtend an die Mantelfläche des Vakuumrohres 22 an, während ein Innenteil 26 der Manschetten 23, 24 gegen den inneren Schlauchabschnitt 2a einwirkt und diesen in Radialrichtung nach außen, d.h. in Richtung zum äußeren Schlauchabschnitt 2b, 2c drückt. Hierdurch entsteht ein nach außen dicht abgeschlossener Raum zwischen dem Vakuumrohr 22, den endseitigen Dichtungsmanschetten 23, 24 und dem äußeren Schlauchabschnitt 2b, 2c des Stülpschlauchs 2, der ferner über die Längsschlitze 6 und die Nuten 5a in der Antriebshülse 5 eine Verbindung zu dem Hohlraum zwischen dem äußeren und inneren Schlauchabschnitt 2a, 2b, 2c hat.

Alternativ zu dieser Ausgestaltung kann das Vakuumrohr 22 auch in zwei Halbschalen längsgeteilt sein, die entweder zusammenklappbar an einer Längskante aneinanderscharniert oder vollständig getrennt sind. Auf diese Weise wird das Einlegen des Stülpschlauchs 2 erleichtert. Desweiteren können die Dichtungsmanschetten 23, 24 durch eine nicht näher gezeigte aktive Spreizvorrichtung (nicht abgebildet) ersetzt werden, mittels der die Stülpbereiche 2e und 2d des Stülpschlauchs 2 gegen das Vakuumrohr 22 gepreßt werden können. Diese Spreizvorrichtung hat ähnlich einer Füllenzange eine Anzahl von (drei) Spreizelementen, die in die Schlauchmündung an den vorderen und hinteren Stülpbereichen 2d, 2e eingeschoben werden. In einem nachfolgenden Arbeitsgang werden diese Zapfen im wesentlichen radial nach außen bewegt, wodurch der Schlauch 2 an die Rohrinnenwandung dichtend angepreßt wird. Die abdichtende Wirkung dieser Spreizvorrichtung kann ferner erhöht werden durch eine an beiden Enden entsprechende beispielsweise kleeblattförmige Innenkontur des Vakuumrohrs, die sicherstellt, daß der Schlauch nicht nur an den Stellen, an denen die Spreizelemente eingreifen, sondern auch dazwischen an der Rohrinnenwandung anliegen.

Schließlich kann das Vakuumrohr 22 auch derart ausgeformt sein, daß es im Bereich der Antriebshülse 5 einen Hohlraum entsprechend dem Gehäuse 11 der Antriebseinrichtung aufweist, so daß das gesamte Stülpschlauchsystem bestehend aus Stülpschlauch, Antriebshülse und Antriebseinrichtung vormontiert in das Vakuumrohr eingelegt werden kann.

Gemäß der Fig. 4 hat das Vakuumrohr 22 ferner einen Leitungsanschluß 27, vorzugsweise im Bereich der Antriebshülse 5, der über eine Leitung mit einer Vakuumpumpe (nicht gezeigt) verbunden ist. Wird die Vakuumpumpe in Betrieb genommen, wird Luft aus dem Hohlraum zwischen dem Vakuumrohr 22 und dem äußeren Schlauchabschnitt 2b, 2c sowie zwischen dem äußeren und inneren Schlauchabschnitt 2a, 2b, 2c über die Öffnung 6 und die Längsnuten 5a der Antriebshülse 5 abgesaugt, wobei sich der innere Schlauchabschnitt 2a gegen den äußeren Schlauchabschnitt 2b, 2c anlegt und dabei den Innendurchmesser des Stülpschlauchs 2 auch im Bereich der vorderen und hinteren Umstülpungen 2d, 2e vergrößert. In den so aufgeweiteten Stülpschlauch 2 läßt sich nunmehr der Endoskopschaft 1 in einfacher Weise einführen, wobei dieser Enführvorgang zusätzlich durch Bestreichen des Schafts 1 mit einem Schmiermittel erleichtert werden kann.

Ist der Endoskopschaft 1 innerhalb des Stülpschlauchs 2 richtig positioniert, wird durch Ausschalten der Vakuumpumpe erneut ein Druckausgleich in den vorstehend genannten Hohlräumen erzeugt, wobei sich der innere Schlauchabschnitt 2a wieder auf dessen ursprünglichen Innendurchmesser zusammenzieht und sich die endseitigen Wülste in den Umstülpbereichen 2d, 2e gegen den Endoskopschaft 1 dichtend anlegen. Mit Entfernen der Dichtungsmanschetten 23, 24 und Abziehen des Vakuumrohrs 22 ist die Montage des Endoskopschafts 1 und des Stülpschlauchs 2 beendet.

Zur Funktions- und Betriebsweise des Endoskops gemäß dem ersten Ausführungsbeispiel der Erfindung wird folgendes ausgeführt:

Zum Einführen des Endoskops, bzw. des Endoskopschafts 1 in den Darm eines Patienten über dessen Anus wird der Antriebsmechanismus, d.h. das Antriebsgehäuse 11 auf einen Sockel montiert, der bezüglich einer Platte, auf der der Patient zumindest teilweise liegt, höhenverstellbar und in jede Richtung schwenkbar ist, so daß das distale Ende 3 des Endoskopschafts 1 entsprechend dem individuellen Korperbau des Patienten angepaßt werden kann. Anschließend wird das distale Ende 3 des Endoskopschafts 1, d.h. ein kurzes Stück in den Anus eingeschoben, solange, bis der Stülpschlauch 2 den Schließmuskel passiert hat. Hierauf wird über den Endoskopschaft 1 bzw. ein darin geführter Kanal CO2-Gas in den Darm eingeleitet, um diesen zu entfalten, worauf die Antriebseinrichtung 10 betätigt wird, um die Reibräder 14 mit einer vorbestimmbaren oder während des Betriebs auch änderbaren Umdrehungszahl anzutreiben. Entsprechend dieser Umdrehungszahl wird der innere Schlauchabschnitt 2a langsam in Eindringrichtung des Endoskops vorgeschoben, wobei er sich im vorderen Umstülpbereich 2d kontinuierlich zu dem vorderen, äußeren Schlauchabschnitt 2b umstülpt und dabei die Darmwandung seitlich auskleiden kann. Gleichzeitig wird die Antriebskraft der Reibräder 14 auch auf den Endoskopschaft 1 übertragen, da durch die Anpreßkraft der Reibräder 14 auf den relativ weichen inneren Stülpschlauchabschnitt 2a dieser auf den Endoskopschaft 1 angedrückt wird und diesen trotz des dazwischen sich ausbreitenden Schmiermittels durch Reibungskräfte mitnimmt. D. h., durch den Vorschub des inneren Schlauchabschnitts 2a wird der Endoskopschaft 1 durch Reibungskräfte zwischen dem Stülpschlauch 2 und dem Schaft 1, gegebenenfalls auch geringfügig durch Druckkräfte in Vorschubrichtung zwischen dem vorderen Umstülpbereich 2d und dem vorderen Klemmstück 7 des Endoskopschafts 1 mitgeschoben und somit in den Darm eingeführt.

Um nunmehr die Vorschubgeschwindigkeit des Endoskopschafts 1 nicht über die Eindringgeschwindigkeit des Stülpschlauchs 2 anwachsen zu lassen, wie bereits in der Beschreibungseinleitung ausführlich erläutert wurde, ist ein Rückhalten oder Abbremsen des Endoskopschafts 2 gegenüber dem inneren Schlauchabschnitt 2a erforderlich. Dies geschieht vorliegend über das hintere Klemmstück 8, an welches sich der hintere Umstülpbereich 2e des Stülpschlauchs 2 anlegt, der sich zwangsläufig mit der gleichen Geschwindigkeit bewegt, wie der vordere Umstülpbereich 2d und der somit die Vorschubgeschwindigkeit des Endoskopschafts 1 bezüglich der Eindringgeschwindigkeit des Stülpschlauchs 2 synchronisiert (d.h. das hintere Klemmstück 8, das fest auf dem Endoskopschafts 1 sitzt, hält diesen zurück und leitet dabei die Reaktionskraft in den hinteren Stülpschlauchabschnitt 2c ein). Die Eindringgeschwindigkeit des Stülpschlauchs 2 ist demzufolge gleich groß wie die des Endoskopschafts 1.

An dieser Stelle sei darauf hingewiesen, daß während des Synchronisationsvorgangs der Vorschubbewegungen des Stülpschlauchs 2 und des Endoskopschafts 1 dieser an den vorderen und hinteren Wülsten wie auch im Bereich der Antriebshülse 5 an dem inneren Stülpschlauchabschnitt 2a gleitet, wobei jedoch die Gleitschwindigkeit des Endoskopschafts 1 bezüglich der vorderen und hinteren Wülste, d.h. an den Umstülpbereichen 2d und 2e bzw. gegenüber dem inneren Schlauchabschnitt 2a lediglich der Relativgeschwindigkeit beider Bauteil entspricht, die nur halb so groß ist, wie die Absolutgeschwindigkeit des inneren Stülpschlauchsabschnitts 2a.

Um hierbei die Bremskräfte auf den hinteren Umstülpbereich 2e nicht zu groß werden zu lassen und damit ggf. ein Zusammenstauchen des Stülpschlauchs 2 im hinteren äußeren Schlauchabschnitt 2c durch die aufzubringenden Bremskräfte zu verhindern, ist es erforderlich, die Reibungskräfte zwischen dem Endoskopschaft 1 und dem inneren Schlauchabschnitt 2a möglichst gering zu halten.

Wie vorstehend bereits beschrieben wurde, bildet sich in dem Ringspalt 9 zwischen dem Schaft 1 und dem inneren Schlauchabschnitt 2a auch im Bereich der Antriebseinrichtung 10 ein nahezu durchgehender Schmierfilm aus, der gerade noch einen Vortrieb des Endoskopschafts 1 im wesentlichen durch die Reibräder 14 ermöglicht, jedoch auch die entstehende Reibung bei der besagten Relativbewegung zwischen den beiden Bauteilen verringert. Darüber hinaus müssen die Umstülpbereiche 2d, 2e und die vorderen und hinteren Klemmstücke 7, 8 aufgrund des geringen Vortriebsanteils auf das vordere Klemmstück 7 bzw. der geringen notwendigen Bremskraft auf das hintere Klemmstück 8 nicht so stark aufeinandergepreßt werden, um beispielsweise eine Dichtwirkung zu erzielen, wie dies bisher im Stand der Technik der Fall war, da dieser Dichtungseffekt bereits durch das Zusammemwirken zwischen den wulstförmigen Umstülpbereichen 2d, 2e und dem Endoskopschaft 2 erreicht wird. Im übrigen wird durch die zweite Schmiermittelfördervorrichtung 19 das Schmiermittel in den Hohlraum zwischen dem inneren und äußeren Schlauchabschnitt 2a, 2b, 2c mit einem vorbestimmten Druck gepreßt, so daß hierdurch die Reibungskräfte weiter verringert werden. Hierbei reicht unter Umständen aufgrund geringer Leckage ein anfänglicher Schmiervorgang zur Befüllung des Hohlraums zwischen dem äußeren und inneren Stülpschlauchabschnitt aus, d.h. daß während zumindest einer Behandlungsdauer nicht unbedingt Schmiermittel nachgepreßt werden muß.

Das Zusammenwirken der Reibräder 14 als ein gemeinsamer kontinuierlicher Antrieb für den Stülpschlauch 2 und für den Endoskopschaft 1, der vorderen und hinteren Wülste als Dichtungen zusammen mit dem Endoskopschaft 1 sowie des durchgehenden Schmierfilms zwischen dem inneren Schlauchabschnitt 2a und dem Schaft 1 erlaubt somit einen äußerst exakten und präzise steuerbaren Vortrieb des Endoskopschafts 1, der dessen Handhabung im Vergleich zum Stand der Technik wesentlich erleichtert.

An dieser Stelle sei darauf hingewiesen, daß an Stelle des hinteren Klemmstücks 8 auch eine zusätzliche externe Synchronisations-Antriebseinrichtung vorgesehen werden kann, die über eine Anzahl von Reibrädern auf den Endoskopschaft 1 an dessen hinterem Endabschnitt einwirkt und somit die kontinuierliche Eindringbewegung des Endoskops gewährleistet.

Des weiteren kann als externer Synchronisations-Antrieb an Stelle der Reibradkonstruktion ein Spindelantrieb für den geregelten kontinuierlichen Vortrieb des Endoskopschafts 1 vorgesehen sein.

Das Zurückziehen des Endoskops erfolgt im wesentlichen auf die gleiche Weise wie der Eindringvorgang, wobei in diesem Fall jedoch das vordere Klemmstück 7 die Synchronisation der Bewegungsgeschwindigkeiten zwischen dem Endoskopschaft 1 und dem Stülpschlauch 2 übernimmt, während das hintere Klemmstück 8 weitestgehend belastungsfrei ist.

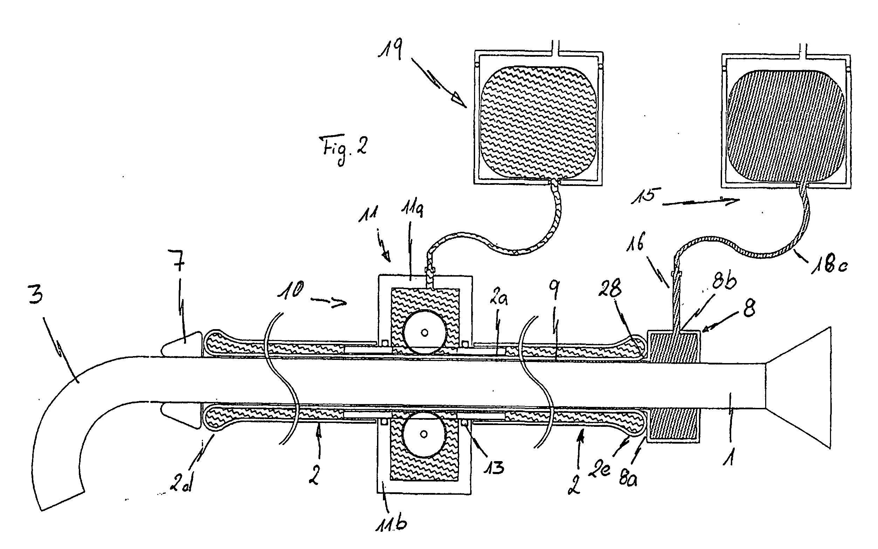

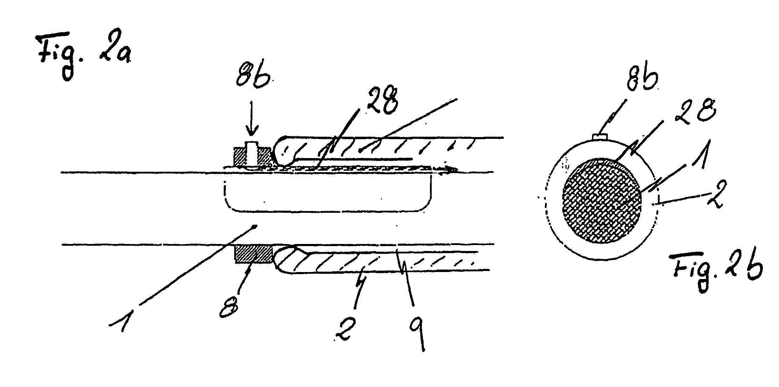

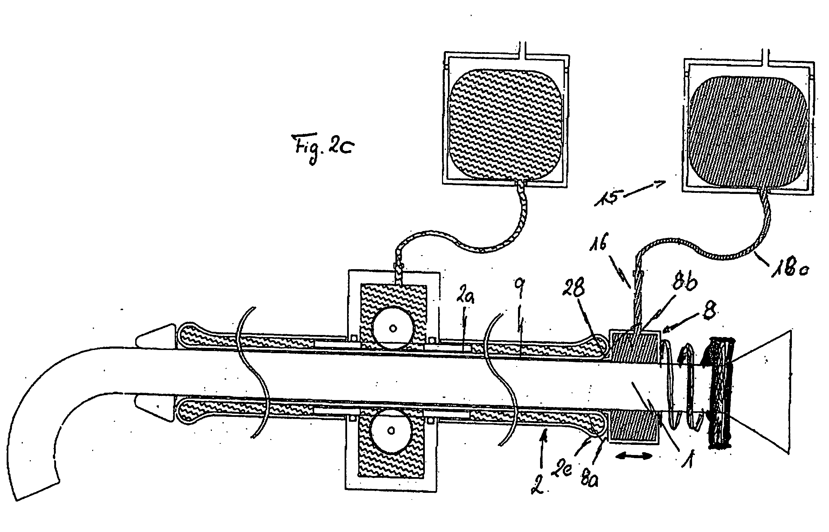

Im nachfolgenden wird ein zweites bevorzugtes Ausführungsbeispiel der Erfindung anhand der Fig. 2, 2a und 2b beschrieben, wobei lediglich auf die zum ersten Ausführungsbeispiel unterschiedlichen Konstruktionsmerkmale eingegangen wird.

Wie in der Fig. 2 gezeigt wird, ist gemäß dem zweiten Ausführungsbeispiel der Erfindung das hintere Klemmstück 8 als eine hohle Manschette ausgeführt, die zusammen mit dem Endoskopschaft 1 einen im wesentlichen geschlossenen Hohlraum ausbildet. Vorzugsweise ist die hintere hohle Manschette axial in zwei Halbschalen symmetrisch teilbar, um so besser an den Endoskopschaft 1 angelegt werden zu können. Die zum Stülpschlauch 2 gerichtete Seiten- oder Anlagewandung 8a des manschettenförmigen Klemmstücks 8 hat im Bereich des Endoskopschafts 1 zumindest einen zum Stülpschlauch 2 vorragenden Schmiermitteleinspritzschuh 28. Dieser Schuh 28 ist insbesondere in Fig. 2b in seinem Querschnitt gezeigt. Demzufolge erstreckt sich der Schmiermitteleinspritzschuh 28 zumindest über einen 1/3-Kreis oder über einen 1/2-Kreis um den Endoskopschaft 1. Vorzugsweise sind zwei diametrisch zueinander liegende Schmiermitteleinspritzschuhe 28 vorgesehen, obgleich in Fig. 2b lediglich ein Schuh gezeigt ist. Gemäß der Fig. 2a kann der vorstehend beschriebene Hohlraum der Einfachheit halber auch durch einen Kanal für das Zuführen von Schmiermittel ersetzt sein. Die Schmiermitteleinspritzschuhe 28 liegen ferner zumindest an ihrem äußersten Endabschnitt elastisch auf der Mantelfläche des Endoskopschafts 1 auf und haben jeweils mindestens einen Schmiermittelkanal, wie in der Fig. 2a gezeigt wird, der sich an dem hinteren Ende (Wurzel) des Schmiermitteleinspritzschuhs 28 in den Hohlraum bzw. den Zuführkanal der Manschette und an seinem vorderen freien Ende in den Ringspalt 9 öffnet.

Alternativ zu dem vorstehend beschriebenen Schmiermitteleinspritzschuh 28 können lediglich auch eine Anzahl von Ausnehmungen oder Einkerbungen (nicht näher gezeigt) in der Seitenwand 8a der Manschette 8 vorgesehen sein, die einen Austrittsspalt oder Spalte zwischen dem Endoskopschaft 1 und der Anlagewandung 8a bilden.

Des weiteren ist das hintere Klemmstück 8 mit einem Leitungsanschluß 8b versehen, der über die Zufuhrleitung 16 und den Auslaßschlauch 18c mit der ersten Schmiermittelfördervorrichtung 15 fluidverbunden ist. Alle weiteren Teile des Endoskops gemäß der Fig. 2 entsprechen denen des ersten Ausführungsbeispiels.

Während des Betriebs des Endoskops gemäß dem zweiten bevorzugten Ausführungsbeispiel der Erfindung fördert die erste Schmiermittelfördervorrichtung 15 das Schmiermittel in den Hohlraum innerhalb des hinteren manschettenförmigen Klemmstücks 8, der sich entsprechend anfüllt. Bei einem bestimmten Druck innerhalb des Hohlraums strömt das Schmiermittel aus dem Schmiermittelkanal innerhalb des Schmiermitteleinspritzschuhs 28 bzw. aus den Spalten in der Anlagewandung 8a aus und dringt in den Ringspalt 9 zwischen dem Endoskopschaft 1 und dem inneren Schlauchabschnitt 2a des Stülpschlauchs 2 zur Schmierung der Relativ-Gleitbewegung beider Bauteile ein. Um eine Leckage am Schmiermitteleinspritzschuh 28 bzw. am Austrittsspalt des hinteren Klemmstücks 8 zu vermeiden, wirkt der hintere Umstülpbereich 2e des Stülpschlauchs 2 als Dichtung, gegen den Schaft 1 und gegen die Anlageseite 8a des hinteren Klemmstücks 8. Auch umschließt der hintere Stülpschlauchbereich der Schmiermitteleinspritzschuh 28 über seine gesamte Länge dichtend ab..

Durch diese Maßnahme erübrigt sich die aufwendige Installation von Kanälen innerhalb des Endoskopschafts 1, wodurch die Handhabung des Endoskops weiter verbessert wird.

Abschließend sei bezüglich des ersten und zweiten Ausführungsbeispiels der Erfindung darauf hingewiesen, daß insbesondere der Antrieb des Endoskops durch Bauteile unterschiedlich zu den Reibrädern 14 erfolgen kann. So könnten beispielsweise für die Vorschubeinrichtung 10 des Stülpschlauchs 2 Zahnräder vorgesehen sein, die auf den inneren Schlauchabschnitt 2a einwirken. All diese Varianten haben jedoch gemeinsam, daß die in Radialrichtung wirkende Anpreßkraft ausreicht, um den inneren Schlauchabschnitt 2a gegen den Endoskopschaft 1 für dessen kontinuierlichen Antrieb trotz des Vorhandenseins von Schmiermittel zu drücken, wodurch die exakte Positionierbarkeit des distalen Endes an einer zu untersuchenden Stelle ermöglicht wird.

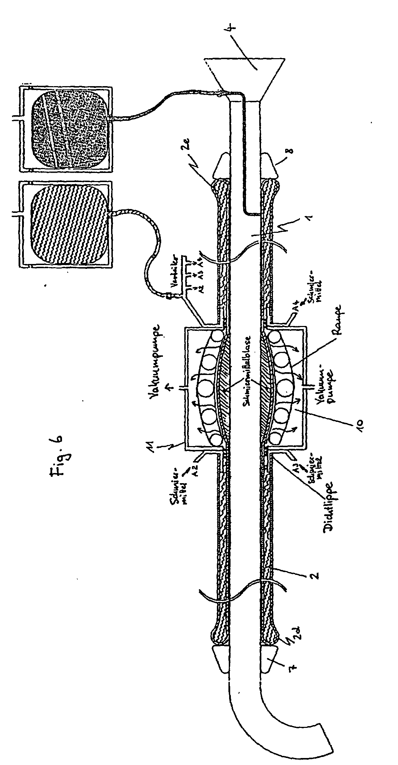

Schließlich wird nachfolgend ein drittes bevorzugtes Ausführungsbeispiel der Erfindung mit Bezug auf die Fig. 5 näher beschrieben, wobei nur auf die Bauteile eingegangen wird, die zu den vorherstehenden Ausführungsbeispielen unterschiedlich sind. Alle weitere Merkmale entsprechen denen des ersten oder zweiten Ausführungsbeispiels.

Gemäß der Fig. 5 besteht die Antriebseinrichtung des dritten Ausführungsbeispiels aus einem Antriebsgehäuse 11, das um die Schlauchführungshülse 5 wie im erst und zweiten Ausführungsbeispiel gelegt ist und in dem zumindest zwei Vakuumgreifer in Axialrichtung des Endoskopschafts 1 alternierend bewegbar angeordnet sind. Die Vakuumgreifer bestehen aus kleinen, durch die Öffnungen in der Antriebshülse 5 durchgreifenden, an dem inneren Stülpschlauchabschnitt 2a dicht anliegenden Klötzchen mit Saugnäpfen, die auf der mit dem Schlauch 2a in Kontakt stehenden Seite der Klötzchen ausgebildet sind und die durch einen von einer Vakuumpumpe erzeugten Unterdruck das Schlauchmaterial im Bereich des inneren Stülpschlauchabschnitts 2a ansaugen und so eine kraftund formschlüssige Verbindung zum inneren Schlauchabschnitt 2a herstellen. Die Vakuumpumpe (nicht gezeigt) ist hierfür an einen Vakuumanschluß angeschlossen, der im Gehäuse 11 der Antriebseinrichtung 10 ausgebildet und über Kanäle innerhalb der Klötzchen mit den Suagnäpfen verbunden ist. Die Bewegung der Klötzchen, welche durch eine nicht näher gezeigte Bewegungsmechanik bewerkstelligt wird, sowie das Erzeugen des Unterdrucks innerhalb der Saugnäpfe wird derart gesteuert, daß die Klötzchen abwechselnd bzw. bei mehr als zwei Klötzchen in gleichmäßigen Abständen fortlaufend mit einsprechend synchronem Ansaugen und Freigeben des inneren Stülpschlauchabschnitts 2a hin- und herbewegt werden und dabei eine quasi kontinuierliche und gleichmäßige Vorschubbewegung des Stülpschlauchs 2 erzeugen.

Wie in der Fig. 5 gut zu erkennen ist, bewirkt das Vakuumisieren der Saugnäpfe ein geringfügiges Abheben des inneren Stülpschlauchabschnitts 2a vom Endoskopschaft. Die Vorschubkraft auf den Endoskopschaft 1 erfolgt in diesem Ausführungsbeispiel im wesentlichen wie bei den bisherigen Ausführungen. Die gesamte Anordnung ist dabei wie im ersten und zweiten Ausführungsbeispiel mit einem Schmiermittel gefüllt, das die Schmierung des Stülpschlauchs 2 übernimmt.

Die Fig. 6 zeigt eine zum dritten Ausführungsbeispiel alternative Ausführungsvariante, die nicht unter den Schutzbereich der Ansprüche fällt, bei der durch Erzeugen eines Unterdrucks eine Antriebskraft auf den Stülpschlauch anlegbar ist.

Wie aus der Fig. 6 zu entnehmen ist, bildet der beidseitig umgestülpte Stülpschlauch 2 zusammen mit einem Gehäuse 11 des Antriebsmechanismus ein einstückiges Bauteil ohne die Anordnung einer Antriebshülse. D.h., daß in diesem Ausführungsbeispiel die freien Enden der äußeren vorderen und hinteren Schlauchabschnitte 2b und 2c direkt an den Seitenwänden des Antriebsgehäuses 11 dichtend befestigt sind.

Der Antriebsmechanismus dieses Ausführungsbeispiels wird dabei durch eine Art Raupenantrieb bestehend aus zumindest einem elastischen Endlosband gebildet, welches über zumindest zwei in Vorschubrichtung voneinander beabstandeter Räder gespannt und angetrieben wird. Die gesamte Antriebseinrichtung ist in dem vorzugsweise in Längsrichtung zweigeteilten, luftdicht verschließbaren Gehäuse untergebracht, welches einen Sauganschluß aufweist. Das Endlosband weist an seiner Oberfläche eine Anzahl von Öffnungen oder eine Perforation auf. Der Sauganschluß ist über eine Schlauchleitung an eine nicht gezeigte Vakuumpumpe angeschlossen.

Das Gehäuse 11 selbst ist auf seiner dem inneren Schlauchabschnitt 2a zugewandten Seite offen, wobei das Endlosband derart geführt ist, daß es die offene Seite des Gehäuses 11 möglichst dicht verschließt. Spalte zwischen den Seitenrändern des Endlosbands und dem Gehäuse 11 können durch geeignete Dichtlippen verschlossen sein, die an den Gehäusewänden befestigt sind und an denen das Endlosband gleitet. Des weiteren ist das Gehäuse 11 bezüglich des daran befestigten Stülpschlauchs 2 derart angeordnet, daß sich die nach außen exponierte Seite des Endlosbands mit einem geringen Spalt oder Abstand oberhalb des inneren Schlauchabschnitts 2a ausrichtet.

Im Betrieb dieser Vorrichtung wird der Innenraum des Gehäuses 11 durch die Vakuumpumpe evakuiert, wobei Luft durch die Perforation in dem Endlosband angesaugt wird. Hierbei wird gleichzeitig der innere Schlauchabschnitt 2a mit angesaugt, der sich dichtend an die Außenseite des Endlosbands anlegt. Durch dieses Abdichten der Perforation erhöht sich der Unterdruck innerhalb des Gehäuses derart, daß sich das Endlosband aufgrund dessen Elastizität nach innen wölbt und dabei den inneren Schlauchabschnitt 2a mitnimmt, wie in der Fig. 6 deutlich gezeigt wird.

Durch diese Maßnahme wird der innere Schlauchabschnitt 2a über die Länge des Endlosbands fest an diesem gehalten und gleichzeitig vom Endoskopschaft 1 beabstandet. Der Vortrieb des Endoskopschafts 1 erfolgt somit im wesentlichen durch die Anlagekräfte, die bei einer Vorschubbewegung des vorderen äußeren Schlauchabschnitts 2b durch das Endlosband am vorderen Umstülpbereich 2d auf das vordere Klemmstück 7 aufgebracht werden, wobei der innere Schlauchabschnitt 2a insbesondere im Bereich des Antriebseinheit kontaktlos bleibt. Aufgrund der geringen Reibungskräfte ist die notwendige Vorschubkraft auf das vordere Klemmstück 7 relativ niedrig, so daß mit keinem Schuppen (Faltenbildung) im vorderen Abschnitt des Stülpschlauchs 2 zu rechnen ist. Im übrigen wird wie beim ersten und zweiten Ausführungsbeispiel ein Schmiermittel in den Ringspalt 9 gepreßt, wodurch die Reibung insbesondere im Bereich der vorderen und hinteren Dichtungswülste 2d, 2e weiter verringert wird.

Auch das dritte Ausführungsbeispiel wie auch dessen alternative Ausführungsvariante sieht demzufolge wie beim ersten und zweiten Ausführungsbeispiel einen kontinuierlich bzw. quasikontinuierlich arbeitenden mechanisch auf den Stülpschlauch 2 einwirkenden Antriebsmechanismus vor, der eine exakt steuerbare Fortbewegung des Endoskops gewährleistet.

Die Erfindung betrifft zusammenfassend ein Stülpschlauchsystem vorzugsweise für ein Endoskop, das einen inneren Schaft hat, der in einem Stülpschlauch der Doppelstülpbauart geführt ist. Der Antrieb des Schlauchs erfolgt über eine Anzahl von Reibrädern, Saugnäpfen oder Raupen, die auf einen inneren Schlauchabschnitt einwirken, um das System für eine kontinuierliche Bewegung anzutreiben. Zur Abdichtung des Spalts zwischen dem Schaft und dem inneren Schlauchabschnitt bilden vordere und hintere Umstülpbereiche jeweils eine Aufwülstung, die sich dichtend an den Schaft anlegen. Die Zufuhr von Schmiermittel in den Spalt zwischen Schaft und innerem Schlauchabschnitt erfolgt über eine im wesentlichen radiale Schaftbohrung im Bereich des Stülpschlauchs, die entweder über den Schafthohlraum oder eine innere Leitung an eine externe Zuführleitung angeschlossen ist oder über ein hinteres Klemmstück mittels eines der Schaftoberfläche angepaßten Schmiermitteleinspritzschuhs.

## Patentansprüche

1. Stülpschlauchsystem mit einem Endoskopschaft (1), der in einem beidseits gestülpten Schlauch (2) gleitend geführt ist, der durch eine Antriebseinrichtung (10) fortbewegbar ist, die auf einen inneren Schlauchabschnitt (2a) des Stülpschlauchs (2) einwirkt, der eine Schlauchführungshülse (5) aus einem steifen Material hat, die über den inneren Schlauchabschnitt (2a) unter Ausbildung eines Zwischenspalts gezogen ist und an deren beiden axialen Endabschnitten die freien Enden äußerer Schlauchabschnitte (2b, 2c) fixiert sind, die durch Umstülpen und Zurückführen des inneren Schlauchabschnitts (2a) an zwei axial beabstandeten Umstülpbereichen (2d, 2e) gebildet werden, **dadurch gekennzeichnet, dass** die Schlauchführungshülse (5) die Befestigungsstelle für die Antriebseinrichtung (10) bildet, wofür die Schlauchführungshülse (5) in ihrem Mittenabschnitt eine Anzahl von Öffnungen (6) hat, durch die Vorschubmittel der Antriebseinrichtung (10) mit dem inneren Schlauchabschnitt in Kontakt bringbar sind.

2. Stülpschlauchsystem nach Anspruch 1, **dadurch gekennzeichnet, dass** die Öffnungen (6) in Form von Längsschlitzen ausgebildet und in gleichem Winkelabstand zueinander angeordnet sind.

3. Stülpschlauchsystem nach Anspruch 2, **dadurch gekennzeichnet, dass** der Stülpschlauch (2) über den inneren Schlauchabschnitt (2a), die äußeren Schlauchabschnitte (2b, 2c) sowie die Hülse (5) einen gegenüber der Umgebung abgedichteten Hohlraum ausbildet, der lediglich über die Öffnungen (6) in der Hülse (5) einen Zugang hat.

4. Stülpschlauchsystem nach einem der Ansprüche 1 bis 3, **dadurch gekennzeichnet, dass** die Hülse (5) an ihrer Innenseite eine Anzahl von im wesentlichen in Axialrichtung verlaufenden Nuten (5a) hat, die sich an den Stirnflächen der Hülse (5) öffnen.

5. Antriebseinrichtung für ein Stülpschlauchsystem mit den Merkmalen gemäß dem Anspruch 1, **gekennzeichnet durch** ein zweigeteiltes, zusammenklappbares Gehäuse (11), welches manschettenförmig um die Hülse (5) des Stülpschlauchs (2) legbar ist und in zusammengeklapptem Zustand mit der Hülse (5) einen nach außen dicht verschlossenen Hohlraum bildet, in welchem Reibräder (14) untergebracht sind, die an dem Gehäuse (11) lagernd **durch** das Zusammenklappen des Gehäuses (11) **durch** die Öffnungen (6) der Hülse (5) gegen den inneren Schlauchabschnitt (2a) preßbar sind.

6. Antriebseinrichtung nach Anspruch 5, **dadurch gekennzeichnet, dass** die Reibräder (14) federnd am Gehäuse (11) gelagert sind, um eine entsprechend der Federkraft vorbestimmte oder über eine zusätzliche Justiereinrichtung einstellbare Anpresskraft auf den inneren Schlauchabschnitt (2a) aufzubringen.

7. Antriebseinrichtung nach einem der Ansprüche 5 und 6, **dadurch gekennzeichnet, dass** die Reibräder (14) an ihren Laufflächen mit einem Antirutschbelag versehen oder ausgebildet sind.

8. Antriebseinrichtung nach einem der Ansprüche 5 bis 7, **dadurch gekennzeichnet, dass** am Gehäuse (11) ein Anschluß für das Zuführen eines Schmiermittels, ausgebildet ist, welches über die Öffnungen (6) der Hülse (5) in den Hohlraum des Stülpschlauchs (2) preßbar ist.

## Claims

1. Roll-back tube system with an endoscope shaft (1) which is slidably guided in a tube (2) rolled back at both ends and which can be moved by means of a drive mechanism (10) which acts on an inner tube section (2a) of the roll-back tube (2), which has a tube-guiding sleeve (5) made of a rigid material, which sleeve is drawn over the inner tube section (2a) to form an intermediate gap, and at the two axial end sections of which the free ends of outer tube sections (2b, 2c) are fixed, which sections are formed by turning back the inner tube section (2a) and bringing it back at two axially spaced turn-back areas (2d, 2e), **characterized in that** the tube-guiding sleeve (5) forms the fastening point for the drive mechanism (10), for which the tube-guiding sleeve (5) has in its central section a number of openings (6), through which advancing means of the drive mechanism (10) can be brought into contact with the inner tube section.

2. Roll-back tube system according to Claim 1, **characterized in that** the openings (6) are designed in the form of longitudinal slots and are arranged at the same angular distance from each other.

3. Roll-back tube system according to Claim 2, **characterized in that** the roll-back tube (2) forms, by way of the inner tube section (2a), the outer tube sections (2b, 2c) and the sleeve (5), a cavity which is sealed off from the environment and can only be accessed via the openings (6) in the sleeve (5).

4. Roll-back tube system according to one of Claims 1 to 3, **characterized in that** the sleeve (5) has, on its inner side, a number of grooves (5a) which extend essentially in the axial direction and open out at the end faces of the sleeve (5).

5. Drive mechanism for a roll-back tube system having the features according to Claim 1, **characterized by** a two-part, fold-up housing (11) which can be placed like a cuff around the sleeve (5) of the roll-back tube (2) and, in the folded-up state, forms, together with the sleeve (5), a cavity which is sealed off tight from the outside and in which friction wheels (14) are accommodated, which wheels, mounted on the housing (11), can be pressed against the inner tube section (2a) through the openings (6) in the sleeve (5) when the housing (11) is folded up.

6. Drive mechanism according to Claim 5, **characterized in that** the friction wheels (14) are spring-mounted on the housing (11) in order to apply to the inner tube section (2a) a pressing force which is predetermined according to the spring force or can be adjusted via an additional adjusting device.

7. Drive mechanism according to one of Claims 5 and 6, **characterized in that** the friction wheels (14) are provided or designed with an anti-slip lining on their running surfaces.

8. Drive mechanism according to one of Claims 5 to 7, **characterized in that** the housing (11) is designed with an attachment for the admission of a lubricant, which can be forced via the openings (6) of the sleeve (5) into the cavity of the roll-back tube (2).

## Revendications

1. Système à manchon inversé avec une tige d'endoscope (1), qui est conduite de façon coulissante dans un manchon (2) retroussé de part et d'autre, qui est déplaçable grâce à un dispositif d'entraînement (10), qui agit sur une section de manchon interne (2a) du manchon inversé (2), qui possède une gaine de guidage de manchon (5) conçue à partir d'une matière rigide, qui est étirée sur la section de manchon interne (2a) du manchon inversé sous la formation d'une fente intermédiaire, et les extrémités libres des sections de manchon externe (2b, 2c) sont fixées sur leurs deux sections d'extrémités axiales, qui sont conçues par retroussement et refoulement de la section du manchon interne (2a) sur deux secteurs retroussés écartés axialement (2d, 2e), **caractérisé en ce que** la gaine de guidage du manchon (5) forme le point de fixation pour le dispositif d'entraînement (10), la gaine de guidage du manchon (5) ayant en sa section médiane un nombre d'ouvertures (6), à travers lesquelles des moyens d'avance du dispositif d'entraînement (10) peuvent être amenés en contact avec la section de manchon interne.

2. Système à manchon inversé selon la revendication 1, **caractérisé en ce que** les ouvertures (6) sont conçues en forme de fissures longitudinales et sont disposées selon un espacement angulaire égal les unes par rapport aux autres.

3. Système à manchon inversé selon la revendication 2, **caractérisé en ce que** le manchon inversé (2) forme via la section de manchon interne (2a), les sections de manchon externe (2b, 2c) ainsi que la gaine de guidage du manchon (5), une cavité étanche par rapport à l'environnement, qui possède uniquement un accès via les ouvertures (6) dans la gaine de guidage du manchon (5).

4. Système à manchon inversé selon l'une quelconque des revendications 1 à 3, **caractérisé en ce que** la gaine de guidage du manchon (5) possède sur sa face interne un certain nombre de rainures (5a) se prolongeant essentiellement en direction axiale qui s'ouvrent sur les surfaces frontales de la gaine de guidage du manchon (5).

5. Dispositif d'entraînement pour un système à manchon inversé avec les caractéristiques selon la revendication 1, **caractérisé par** un boîtier (11) divisé et replié qui peut être plié en forme de bague autour de la gaine de guidage du manchon (5) du manchon inversé (2) et forme avec la gaine de guidage du manchon (5), dans un état replié, une cavité fermée hermétiquement vers l'extérieur, dans laquelle des volants de serrage (14) sont logés, qui peuvent être pressés sur le boîtier (11) de façon étendue par le repli du boîtier (11) à travers les ouvertures (6) de la gaine de guidage du manchon (5) contre la section de manchon interne (2a).

6. Dispositif d'entraînement selon la revendication 5, **caractérisé en ce que** les volants de serrage (14) sont stockées par ressort sur le boîtier (11), pour appliquer une force de pression prédéterminée conformément à la force de ressort ou pouvant être réglée via un dispositif de réglage supplémentaire sur la section de manchon interne (2a).

7. Dispositif d'entraînement selon l'une quelconque des revendications 5 et 6, **caractérisé en ce que** les volants de serrage (14) sont prévus ou conçus sur leurs surfaces de roulement avec un revêtement anti-frottement.

8. Dispositif d'entraînement selon l'une quelconque des revendications 5 à 7, **caractérisé en ce qu'**un raccordement est conçu sur le boîtier (11) pour l'alimentation d'un lubrifiant, lequel peut être pressé via les ouvertures (6) de la gaine de guidage du manchon (5) dans la cavité du manchon inversé (2).
